# EUROPEAN PATENT APPLICATION

(11) **EP 0 667 521 A1**
(43) Date of publication of application: **16.08.1995**
(21) Application number: 94925015.3
(22) Date of filing: 26.08.1994
(51) Int. Cl.: G01N 27/327, G01N 27/416

(54) **BOD SENSOR AND BOD MEASURING METHOD**

(30) Priority: 26.08.1993 JP 211954/93
(71) Applicant: AKEBONO BRAKE INDUSTRY CO., LTD., Chuo-ku Tokyo 103 (JP); AKEBONO RESEARCH AND DEVELOPMENT CENTRE LTD., Hanyu-shi Saitama-ken 348 (JP); Karube, Isao, Kawasaki-shi, Kanagawa-ken (JP)
(72) Inventor: KARUBE, Isao, Kawasaki-shi, Kanagawa 216 (JP); YANO, Kiyoko, Meguro-ku, Tokyo 153 (JP)
(74) Representative: Bannerman, David Gardner
(86) International application number: JP9401418
(87) International publication number: WO9506242

(57) **Abstract**

An electrode made of a metal or carbon, a working electrode (20) in contact with this electrode and of a microorganic germ-containing thin film, a counter electrode (7), and a reference electrode (5) as necessary are immersed in an organism-containing solution (30), and the BOD of this solution is measured by determining the quantity of an electric current flowing between the working electrode (20) and counter-electrode (7) or reference electrode (5), applying a voltage between the electrodes by a potentiostat (6). By this method, the concentrations of organic compounds can be measured directly without using an oxygen electrode even when the quantity of dissolved oxygen is small.

## Description

### Field of the Invention

The present invention relates to a BOD sensor. More specifically, the present invention relates to a BOD sensor which does not use an oxygen electrode.

### Background of the Invention

BOD (biochemical oxygen demand) is an important item by which to control the quality of river water and industrial waste water, and is deemed to be an international index of organic water pollution. The water pollution due to organic compounds is degraded or eliminated by the oxidation reaction with aerobic microorganisms, and dissolved oxygen is consumed according to the concentration of the organic compounds. The water pollution is determined by measuring the amount of oxygen consumed. That is, BOD indicates the concentration of organic compounds indirectly in terms of the amount of oxygen decreased.

A method for measuring BOD is regulated by the Japanese Industrial Standard (JIS). However, this method requires an intricate operation, and it takes 5 days to measure BOD by this method. Thus, a rapid, simple method which can measure BOD in an on-line system has been in demand, and a BOD sensor has already been developed and has been utilized to measure industrial waste water or the like.

The BOD sensor which has been used so far comprises a microorganism membrane and an oxygen electrode, and measures BOD in terms of an amount of dissolved oxygen reduced in a solution. As this type of sensor, for example, a microorganism electrode is known in which microorganism metabolizing organic compounds and consuming oxygen is enclosed between a diaphragm of an oxygen electrode and a dialysis membrane covering the diaphragm (Japanese Laid-Open Patent Application (Kokai) No. 54-47,699).

However, it is difficult to measure precisely the value with a waste water containing a small amount of dissolved oxygen by means of the above-mentioned sensor which measures BOD with the amount of dissolved oxygen decreased. Further, since the oxygen electrode contains an electrolyte and the like therein, a sensor requires a certain size.

Under these circumstances, the present invention aims to provide a BOD sensor which can measure the concentration of organic compounds directly without the use of an oxygen electrode and even at small amounts of dissolved oxygen.

### Detailed Description of the Invention

In order to solve the above-mentioned problems, the present inventors constructed a BOD sensor having the following structure.

That is, the present invention is to provide a BOD sensor which comprises a working electrode (microorganism electrode) having an electrode made of metal or carbon and a membrane containing microorganism which membrane is in contact with the electrode, and a counter electrode. Further, the present invention is to provide a method for measuring BOD, which comprises immersing into a solution containing organic compounds a BOD sensor which comprises a working electrode (microorganism electrode) having an electrode made of metal or carbon and a membrane containing microorganism cells which membrane is in contact with the electrode, and a counter electrode; and measuring a current that flows when a potential difference is applied between the microorganism electrode and the counter electrode to measure the BOD of the solution.

Still further, the present invention is to provide a BOD sensor which further comprises, in addition to the above-mentioned working electrode and counter electrode, a reference electrode, and a method for measuring BOD, which further comprises immersing the BOD sensor in a solution containing organic compounds and measuring a current that flows when a potential difference is applied between the working electrode and the reference electrode to measure the BOD of the solution.

The present invention will be described in more detail below.

### 1. BOD sensor of the present invention

The BOD sensor of the present invention comprises a working electrode having an electrode made of metal or carbon (hereinafter simply referred to as "metal electrode") and a membrane which contains microorganism cells and which is in contact with the metal electrode (hereinafter simply referred to as a "microorganism membrane"), and a counter electrode. A BOD sensor of another embodiment of the present invention comprises the working electrode having the metal electrode and the microorganism membrane, the counter electrode, and further a reference electrode.

When a microorganism is present in a solution containing organic compounds, the microorganism metabolizes the organic compounds to acquire energy. During the metabolysis, transfer of electrons occurs in the electron transport system of its respiratory system. At this time, the amount of the organic compounds metabolized correlates to the amount of electron that is transferred. Therefore, the concentration of the organic compounds present around the microorganism is determined by measuring the amount of electrons that transfer, thereby making it possible to measure BOD. It is difficult to measure directly the amount of electron that is transferred. Accordingly, in the present invention, a BOD sensor having the above-mentioned structure is immersed in a solution containing organic compounds, and a certain potential difference is applied between the working electrode and the counter electrode or the reference electrode of the sensor such that the electrons easily transfer to measure the current that flows between both the electrodes. The structure of the BOD sensor of the present invention will be described in more detail below.

The microorganism membrane, for making the microorganism cells present on or near the surface of the metal electrode, can take any form as long as it is thin. For example, a three-dimensional crosslinked structure that includes the microorganism cells therein is applicable. Examples of the three-dimensional crosslinked structure include an alginate gel membrane, an agarose gel membrane, a photo-crosslinkable polyvinyl alcohol membrane and a polyacrylamide membrane. The microorganism may be in membranous form held between the metal electrode and a membrane that does not permeate the microorganism cells such as a dialysis membrane. Further, the microorganism may be immobilized with a polymer membrane. Still further, the microorganism may be immobilized with glutaraldehyde or the like in membranous form on the surface of the metal membrane making up the working electrode. It is preferable that the microorganism in the membrane be alive.

Any kind of microorganism may be available if the transfer of electron occurs in the electron transport system by metabolizing the organic compounds. A procaryote and a eucaryote are both available. However, since the electron transport with the respiratory chain is conducted at the mitochondria, the amount of electrons that transfer to the working electrode is relatively small. For this reason, the procaryote is preferable from the standpoint of sensitivity in the BOD measurement. Examples of procaryotes include Escherichia coli, bacteria of belonging to the genus Bacillus, genus Acinetobacter, genus Gluconobacter and genus Pseudomonas, and actinomycetes. Examples of the eucaryote include a yeast of genus Trichosporon.

The electrode made of metal or carbon receives electrons that are generated by metabolizing the organic compounds of the BOD measurement sample with the microorganism. The working electrode may be made of any materials which are stable, highly electroconductive and substantially harmless to the microorganism. Examples of these materials include metals such as platinum, gold and silver and carbonaceous materials such as graphite and carbon. The shape of the electrode is not particularly limited. The electrode can take the form of a bar, a cylinder or a sheet.

The working electrode constituting the BOD sensor of the present invention is composed of the metal electrode and the microorganism membrane in contact with the metal electrode. When the metal electrode is spaced too far apart from the microorganism membrane, the electrons that are generated from the microorganism membrane cannot transfer to the metal electrode. In this sense, the word "contact" does not necessarily mean that the metal electrode is in complete contact with the microorganism membrane. These electrodes may be close to each other to such an extent that electrons can be transferred in the solution.

Examples of the structure of the working electrode include a structure in which the microorganism is immobilized with the surface of the metal electrode via functional groups, a structure in which a gel membrane containing the microorganism adheres to the metal electrode, and a structure in which the end of the metal electrode is covered with a dialysis membrane and the microorganism is put between the metal electrode and the dialysis membrane. It is also advisable that a microorganism suspension be suction-filtered on a membrane of acetylcellulose to collect the microorganism in membranous form on the membrane and the metal electrode be covered with the dialysis membrane from outside the acetylcellulose membrane. It is further advisable that the metal electrode and the microorganism membrane take such forms that their contact areas are large.

The BOD sensor of the present invention comprises the above-mentioned working electrode and counter electrode and, if required, further the reference electrode. Examples of the material of the counter electrode include platinum, silver, gold and carbon. When the BOD sensor is immersed in the measurement sample solution and the potential difference is applied between the working electrode and the counter electrode, the concentration of the reaction materials on the surfaces of the electrodes decreases and the concentration of the product increases as the reaction of the electrode proceeds. Thus, the potential of the electrode sometimes deviates from the set potential. Accordingly, it is advisable that a reference electrode such as an Ag/AgCI electrode or the like be immersed in a sample solution and the potential of the working electrode be set on the basis of the reference electrode (3-electrode method).

In the BOD sensor of the present invention, the working electrode and the counter electrode and optionally the reference electrode may be formed separately or integrally.

### 2. Method for measuring BOD

In the method for measuring BOD in the present invention, the above-mentioned BOD sensor is immersed in a solution containing organic compounds (measurement sample solution), and the current that flows between the working electrode and the counter electrode or the reference electrode when the potential difference is applied is measured, to measure BOD of the solution thereby.

To be concrete, for example, the above-mentioned BOD sensor is immersed in the measurement sample solution, the potential difference is applied between the working electrode and the counter electrode, and the current that flows between both of the electrodes is measured. In the 3- electrode method, the potential difference is applied between the working electrode and the reference electrode, and a current that flows therebetween is measured. The application of the potential difference and the measurement of the current may be conducted with a potentiostat.

It is preferable to add a mediator to the sample solution, since BOD can be measured at a higher sensitivity. The mediator may be contained in the microorganism membrane or between the microorganism membrane and the metal electrode.

The mediator helps the electrons generating from the metabolism of the organic compounds by the microorganism to transfer to the metal electrode. Any kind of mediators may be available, if it expedites the transfer of electron from the microorganism to the metal electrode. Examples of the mediator include pigments such as 1-methoxy-5-methylphenazinium methylsulfonate (1-M-PMS), 2,6-dichloroindophenol (DCIP), 9-dimethylaminobenzo-a-phenazoxonium chloride, methylene blue, indigotrisulfonic acid, phenosafranin, thionine, new methylene blue, 2,6-dichlorophenol, indophenol, azure B, N,N,N',N'- tetramethyl-p-phenylenediamine dihydrochloride, rezorufine, safranin, sodium anthraquinone ,8-sulfonate, and indigo carmine; biological oxida- tion/reduction materials such as riboflavin, L-ascorbic acid, flavin adenine dinucleotide, flavin mononucleotide, nicotine adenine dinucleotide, lumich- rome, ubiquinone, hydroquinone, 2,6-dichloroben- zoquinone, 2-methylbenzoquinone, 2,5-dihydrox- ybenzoquinone, 2-hydroxy-1,4-naphthoquinone, glutathione, peroxidase, cytochrome C, and fer- rodoxins or their derivatives; and Fe-EDTA, Mn-EDTA, Zn-EDTA, mesosulfate, 2,3,5,6-tetramethyl- p-phenylenediamine, and potassium ferricyanide. The concentration of the mediator is preferably at least about 40 nM.

Of the above compounds, 1-M-PMS, DCIP, potassium ferricyanide and 9-dimethylaminobenzo-a-phenazoxonium chloride are preferable.

BOD is measured by measuring the current flowing between the counter electrode or the reference electrode and the working electrode with the use of a buffer solution free of organic compounds, then measuring likewise a current in the same way with the use of a measuring sample or a measuring sample solution diluted with the buffer solution, and comparing the difference between these currents with the difference between the currents given when a standard sample is used.

The obtained current is dependent on the kind of the microorganism, the contact area between the metal electrode and the microorganism membrane, the kind and the concentration of the mediator, the potential difference applied between the counter electrode and the metal electrode, or the concentration of BOD. Therefore, the current may be set appropriately by conducting a preliminary experiment.

When the BOD sensor of the present invention is immersed in a solution containing the organic compounds, the organic compounds is metabolized by the microorganism in the microorganism membrane of the sensor. Consequently, electrons transfer via the electron transport system. When the potential difference is applied between the counter electrode and the metal electrode, electron transfers from the microorganism membrane to the metal electrode. As a result, the current obtained is different from that given when electrons are not generated. The concentration of the organic compounds, that is, BOD can be measured by measuring the current.

When the mediator is added to the sample solution or to the microorganism membrane at this time, it helps the electrons to transfer from the microorganism to the metal electrode. Accordingly, the measurement sensitivity improves.

### Brief Description of the Drawings

Fig. 1 is a sectional view showing one embodiment of a working electrode that constitutes a BOD sensor of the present invention.
Fig. 2 is a front view showing an example of a BOD-measuring system that uses the BOD sensor of the present invention.
Fig. 3 is a graph showing the relation between a potential applied and the current value.
Fig. 4 is a graph showing the relation between the concentration of a buffer solution and the current value.
Fig. 5 is a graph showing the relation between the concentration of a mediator (DCIP) and the current value.
Fig. 6 is a graph showing the relation between the concentration of a mediator (1-M-PMS) and the current value.
Fig. 7 is a graph showing the relation between BOD and the current value when the type of the mediator is changed.
Fig. 8 is a graph showing an example of the calibration curve when BOD is measured.
Fig. 9 is a graph showing the influence of a dissolved gas on the measurement of BOD.
Fig. 10 is a graph showing the relation between BOD and the current value when 8 mM potassium ferricyanade is used as a mediator.
Fig. 11 is a graph showing the relation between BOD and the current value when 40 nM 9-dimethylaminobenzo-a-phenazoxonium chloride is used as the mediator.
Fig. 12 is a graph showing the relation between BOD and the current value when 80 nM 1-M-PMS is used as the mediator.
Fig. 13 is a graph showing the relation between BOD and the current value when the material of the electrode is changed.
Fig. 14 is a graph showing the relation between BOD and the current value when Escherichia coli is used as the microorganism of the microorganism membrane.
Fig. 15 is a graph showing the relation between BOD and the current value when Bacillus stearothermophilus is used as the microorganism of the microorganism membrane.
Fig. 16 is a graph showing the relation between BOD and the current value when Acinetobacter calcoaceticus is used as the microorganism of the microorganism membrane.
Fig. 17 is a graph showing the relation between BOD and the current value when Trichosporon cutaneum is used as the microorganism of the microorganism membrane.
Fig. 18 is a graph showing the relation between BOD and the current value when Gluconobacter suboxydans is used as the microorganism of the microorganism membrane.
Fig. 19 is a graph showing the relation between BOD and the current value when Pseudomonas aeruginosa is used as the microorganism of the microorganism membrane.
Fig. 20 is a graph showing the influence of a small amount of dissolved oxygen on BOD measurement.
Fig. 21 is a graph showing the relation between BOD and the current value when BOD is measured with a BOD sensor in which microorganism cells are immobilized on a metal electrode with agarose.
Fig. 22 is a graph showing the relation between BOD and the current value when BOD is measured with a BOD sensor in which microorganism cells are immobilized on a metal electrode with photo-crosslinkable polyvinyl alcohol (PVA-SbQ: polyvinyl alcohol stilbazole quaternized).
Fig. 23 is a graph showing the relation between BOD and the current value when BOD is measured with a BOD sensor in which microorganism cells are immobilized on a metal electrode with glutaraldehyde.

### Description of the Preferred Embodiment

The present invention will be described specifically hereinafter.

### Example 1

### BOD sensor

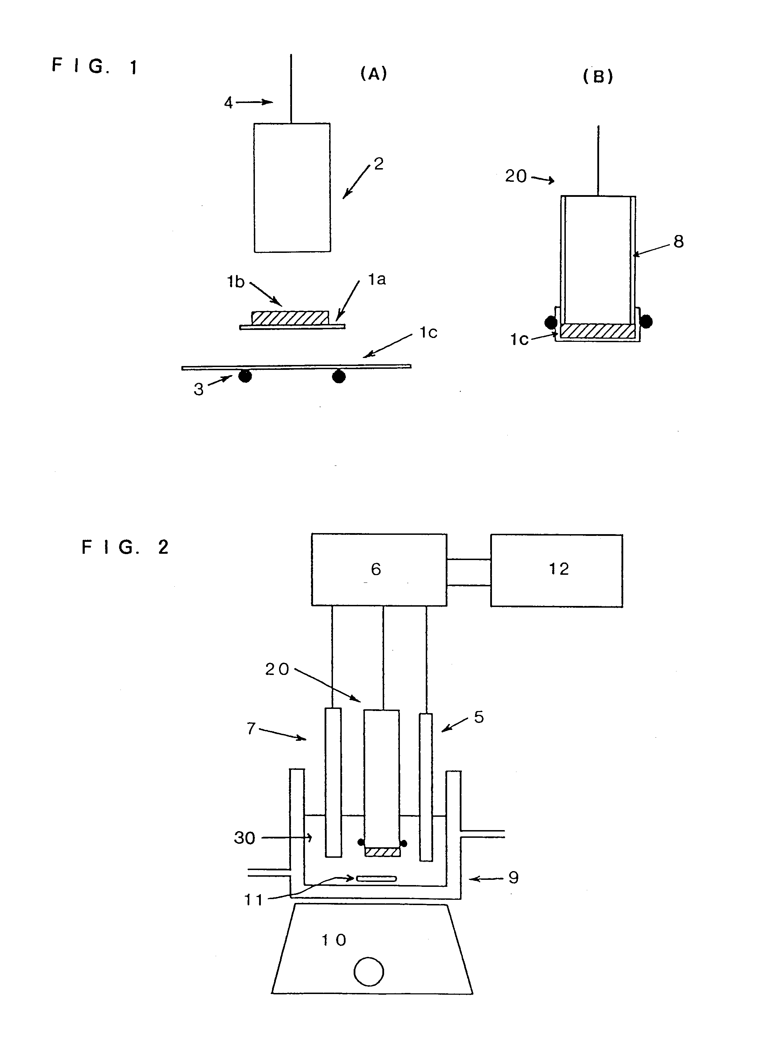

First, an example of a working electrode constituting a BOD sensor and a measuring system that uses the BOD sensor having the working electrode are described on the basis of Figs. 1 and 2.

A microorganism (named "L-GL3") of genus Bacillus which was isolated from an activated sludge was inoculated in 80 ml of a culture medium (pH 7.0) containing 0.3 % K₂HP0₄, 0.1 % KH₂P0₄, 0.03 % MgSO₄·7H₂O, 0.5 % ammonium sulfate, 0.001 % L-glutamic acid and 0.1 % yeast extract, and cultivated at 30 _{°} C for 48 hours with shaking. The culture was centrifuged to separate the cells from the medium. The cells was washed with a 0.05 M phosphate buffer solution (pH 7.0), and suspended in the same buffer solution.

The above-obtained cell suspension was filtered with a porous acetylcellulose membrane 1 a (pore diameter 0.45 am), and cells 1 are adhered to the surface of the membrane. The resulting membrane 1, as a microorganism membrane, was put on one end of a cylindrical platinum electrode 2 having a diameter of 3 mm which the side surfaces were insulated by covering with a Teflon tube 8, such that the surface to which the cells 1 b of the membrane were adhered was in contact with the end of the platinum electrode 2. Then, the above microorganism membrane was further covered with a dialysis membrane 1 c, and fixed with an O-ring 3 (Fig. 1). A lead wire 4 was connected with the other end of the platinum electrode 2 by adhering them with a silver paste. The lead wire may be fixed on the metal electrode by means of a clip or the like. The connecting site of the lead wire and the metal electrode was insulated by covering it with a heat- shrinkable tube. In the following Examples, the insulation of the connecting site between the side surface of the metal electrode and the lead wire was also conducted in the same manner as mentioned above.

Using the working electrode 20 having the above-mentioned structure, BOD was measured according to the below-mentioned measuring system (3-electrode system method) as shown in Fig. 2. The above-mentioned working electrode, a reference electrode 5 (Ag/AgCI electrode) and the counter electrode (platinum electrode) 7 were connected with a potentiostat 6, and immersed in a sample vessel 9 filled with a buffer solution to give a BOD sensor. A fixed potential was applied between the reference electrode and the working electrode, and the current that flowed therebetween was measured. Then, a BOD standard solution or an L-glutamic acid solution was added, and the current value was measured in the same way. During the measurement of BOD, the sample solution was stirred with a magnetic stirrer 10. The measured current value was recorded with a recorder 12.

### Example 2

### Examination at an Optimum Potential

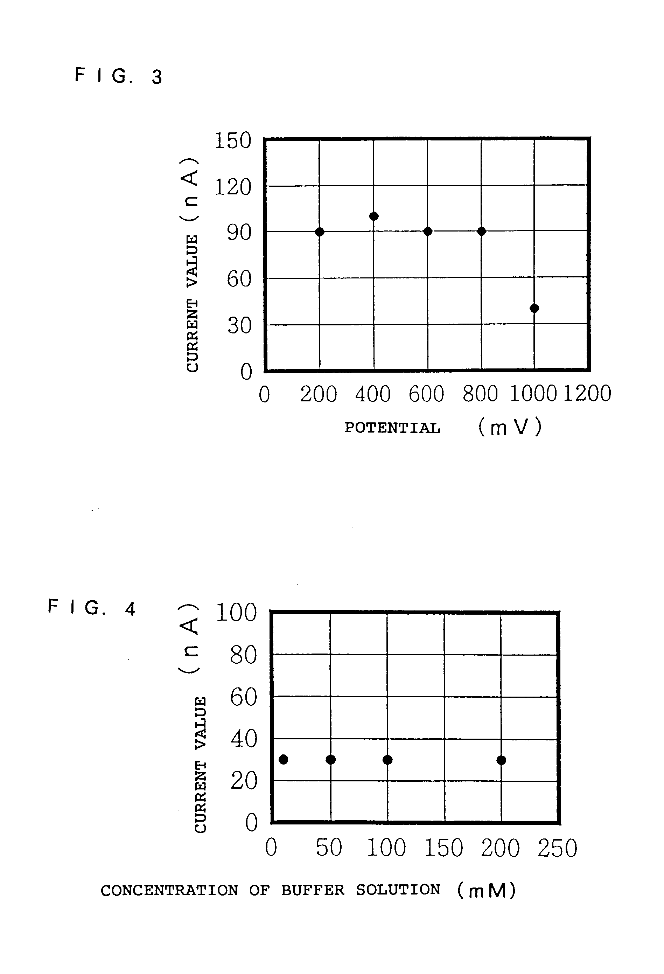

An optimum potential applied to the above-mentioned measuring system was examined. As a sample solution 30, a 0.01 M phosphate buffer solution (pH 7.0) containing 40 nM DCIP and 40 nM 1-M-PMS was used. The BOD sensor was immersed in a sample vessel filled with 5 ml of this buffer solution. A potential at from +200 mV to 1 V was applied, and 40 u.1 of organic compound (150 mg/I L-glutamic acid) was added. Thus, an increase in the current value was measured. The results are shown in Fig. 3.

From the results, it has become apparent that almost equal response are shown at the potential applied from +200 mV to +800 mV and that under the above-mentioned conditions, it is desirous to conduct the measurement within the above range.

### Example 3

### Examination at an optimum concentration of a buffer solution

The concentration of the buffer solution was examined. The increase in the current value was measured in the same manner as in Example 2 except that the concentration of the phosphate buffer solution (pH 7.0) was changed from 10 mM to 200 mM and the potential applied was fixed at +400 mV. The results are shown in Fig. 4.

From the results, it has become apparent that equal response are shown with the phosphate buffer solution (pH 7.0) in the amount of from 10 mM to 200 mM and the measurement can be conducted at least in this amount.

### Example 4

### (1) Examination at an optimum concentration of the mediator

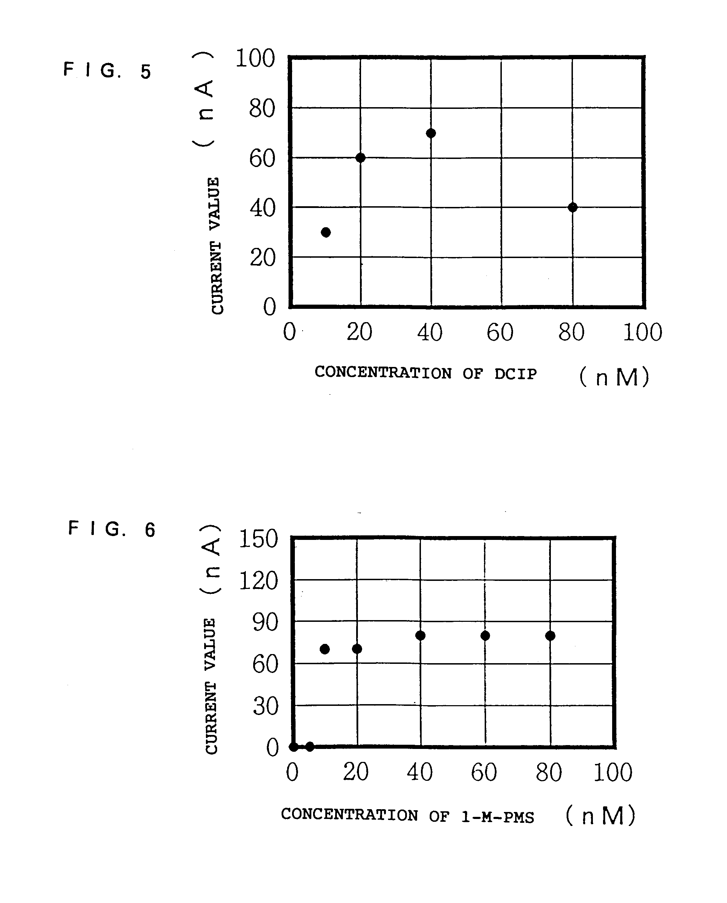

Using DCIP and 1-M-PMS as mediators, the optimum concentration of DCIP was examined when the concentration of 1-M-PMS was 40 nM. An increase in a current value was measured in the same manner as in Example 2 except that the concentration of the phosphate buffer solution (pH 7.0) was 0.01 M, the potential applied was fixed at +400 mV and the concentration of DCIP was varied from 10 nM to 80 nM. The results are shown in Fig. 5. Good response values were obtained when the concentration of DCIP was at least 10 nM.

### Example 5

### (2) Examination at an optimum concentration of a mediator

Using DCIP and 1-M-PMS as mediators, the optimum concentration of 1-M-PMS was examined when the concentration of DCIP was 40 nM. The increase in the current value was measured in the same manner as in Example 4 except that the concentration of DCIP was 40 nM and the concentration of 1-M-PMS was varied from 0 nM to 80 nM. The results are shown in Fig. 6.

From the results, it has become apparent that good response values were obtained when the concentration of 1-M-PMS was at least 10 nM.

### Example 6

### Examination with respect to an influence of the mediator on the current value

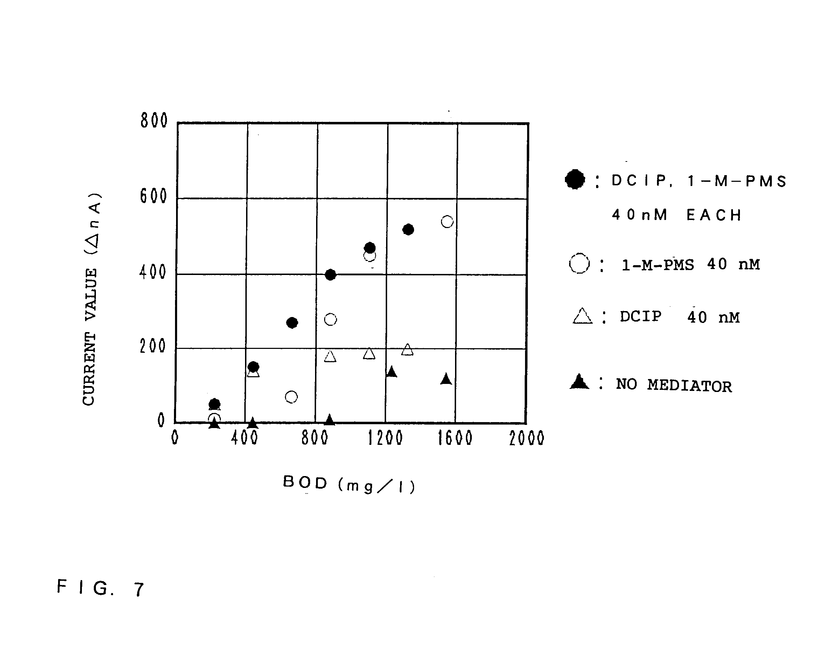

To 5 ml of a 0.01 M phosphate buffer solution (pH 7.0) was added 40 nM 1-M-PMS alone, or 40 nM DCIP alone, or a combination of 40 nM 1-M-PMS and 40 nM DCIP as the mediator. A BOD sensor was immersed in the buffer solution or a buffer solution free of the mediator. A BOD standard solution (a mixed solution containing 150 mg/I of glucose and 150 mg/I of L-glutamic acid (BOD 220 mg/I): hereinafter simply referred to as a "BOD standard solution") was further added, and the current was measured. The results are shown in Fig. 7.

With the addition of a high concentration of the BOD standard solution, the current value was increased, though small, even in the absence of the mediator. However, the addition of the mediator gave a great increase in the current value. Accordingly, it was shown that although BOD can be measured in the absence of a mediator, better results are given with the addition of a mediator.

### Example 7

### Formation of a calibration curve for measuring BOD

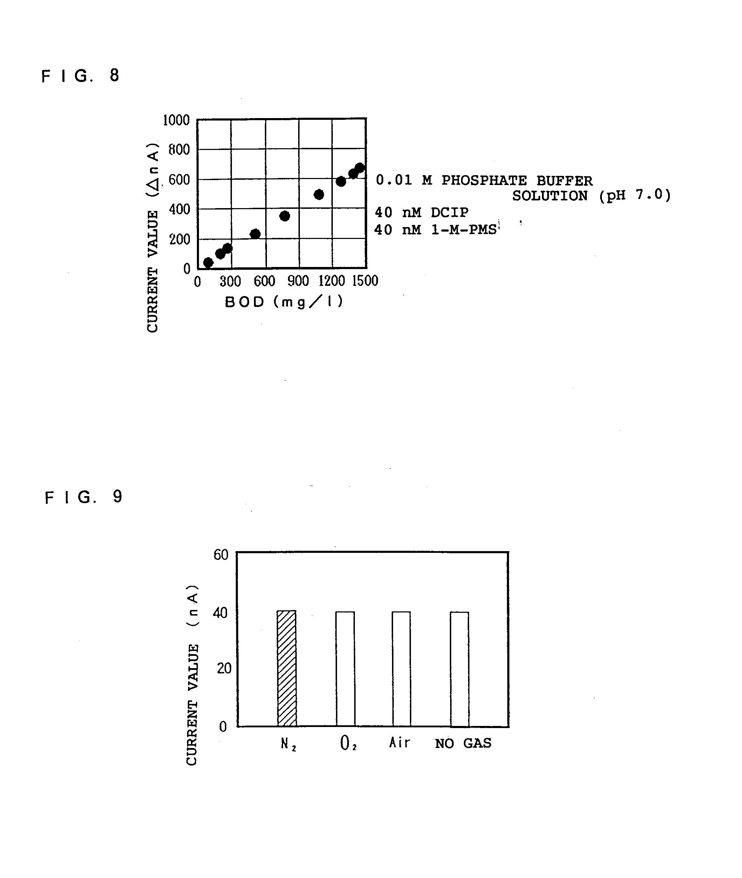

A BOD sensor was immersed in a sample vessel filled with 5 ml of a 0.01 M phosphate buffer solution containing 40 nM DCIP and 40 nM 1-M-PMS. A potential of +400 mV was applied, and a BOD standard solution was added. An increase in the current value was measured. The results are shown in Fig. 8.

From the results, it has become apparent that with the concentration of BOD up to at least approximately 1,500 mg/ml, the current value increases in proportion to the concentration of BOD and BOD can thus be precisely measured by the method of the present invention.

### Example 8

### Examination with respect to the influence of dissolved oxygen on the method of the present invention

The influence of dissolved oxygen on the method of the present invention was examined. Nitrogen, oxygen and air were separately flowed through 5 ml of a 0.01 M phosphate buffer solution (pH 7.0) for 30 minutes, and DCIP and 1-M-PMS were added thereto such that the final concentration of each of DCIP and 1-M-PMS became 40 nM. A BOD sensor was immersed in the mixture. A potential of + 400 mV was applied thereto, and 40 µl of a BOD standard solution was added. An increase in the current value was measured. Likewise, the measurement was conducted without passing the gas. The results are shown in Fig. 9.

Regardless of the passage of the various gases, the current value was constant, and the BOD sensor of the present invention could measure BOD without being influenced by dissolved oxygen. It proves that the BOD sensor of the present invention, unlike the conventional BOD sensor, does not measure BOD with the amount of dissolved oxygen decreased but measures BOD upon measuring the electrons that are generated by metabolizing organic compounds.

### Example 9

### Examination based on other mediators

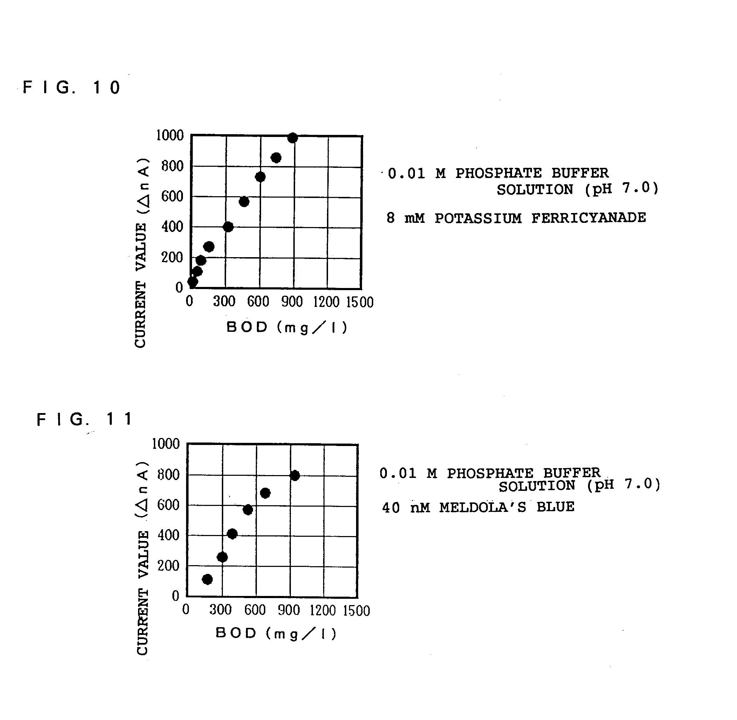

To 5 ml of a 0.01 M phosphate buffer solution (pH 7.0) was added 80 nM 1-M-PMS, 8 mM potassium ferricyanade or 40 nM 9-dimethylaminobenzo- a-phenazoxonium chloride, separately. Then, a BOD sensor was immersed into the solution, and a BOD standard solution was added. The current was then measured. The results are shown in Figs. 10 to 12.

### Example 10

### Examination based on a material of an electrode

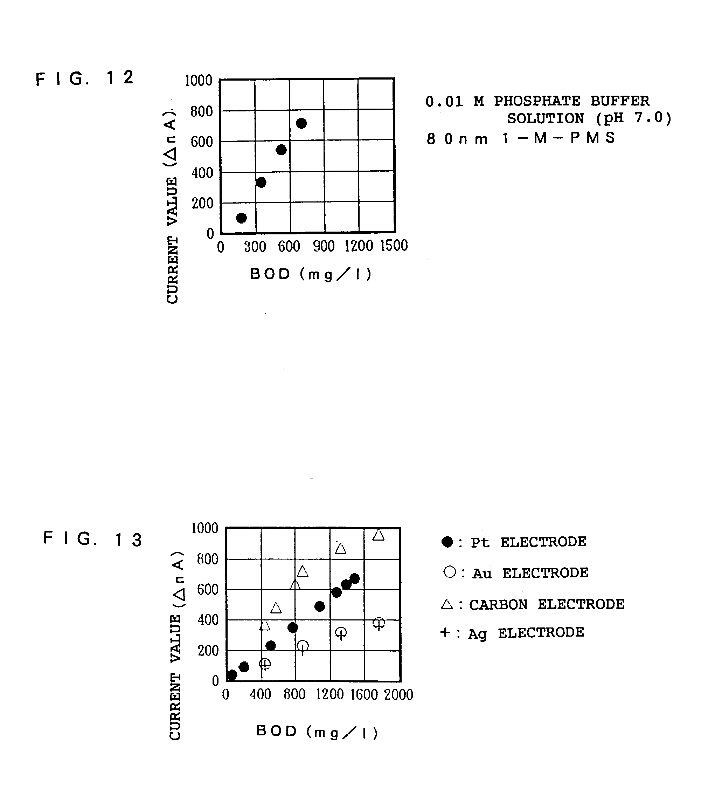

BOD sensors were prepared using a platinum electrode, a gold electrode, a silver electrode and a carbon electrode, the diameter of these electrodes being 3 mm, and BOD was measured in the same manner as in Example 6. At this time, a reference electrode was not used. A potential difference was applied between the working electrode and the counter electrode, and the current that flowed through both of the electrodes was measured. The results are shown in Fig. 13. As is apparent, the same results are obtained with the platinum, gold, silver and carbon electrodes, and BOD can be measured with electrodes of stable metals or electroconductive materials such as carbon.

### Example 11

### Examination based on the microorganism used in a microorganism membrance

### (1) Escherichia coli

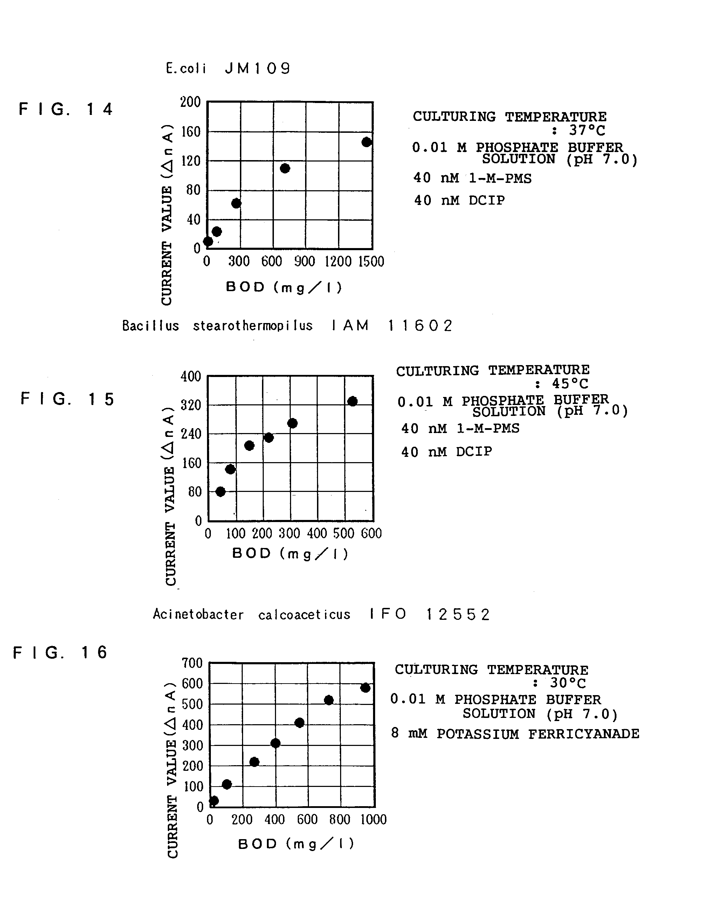

Escherichia coli JM109 was cultivated at 37°C for 12 hours with shaking, and immobilized on a nitrocellulose membrane. The thus-obtained membrane was mounted on a Pt electrode. A BOD sensor comprising the obtained working electrode, a counter electrode and a reference electrode was immersed in a buffer solution containing a mediator (40 nM 1-M-PMS and 40 nM DCIP), and a BOD standard solution was added thereto. An increase in the current value was measured in the same manner as mentioned above. The results are shown in Fig. 14.

### (2) Bacillus stearothermophilus

Bacillus stearothermophilus IAM11602 was cultivated at 45 _{°} C while shaking, and the experiment was conducted in the same manner as in the above (1). The results are shown in Fig. 15.

### (3) Acinetobacter calcoaceticus

Acinetobacter calcoaceticus IF012552 was cultivated at 30 °C, and the experiment was conducted in the same manner as in the above (1). Potassium ferricyanade (8 mM) was used as a mediator. The results are shown in Fig. 16.

### (4) Trichosporon cutaneum

Trichosporon cutaneum IF010466 was cultivated at 30 _{°} C, and the experiment was conducted in the same manner as in the above (1). 1-M-PMS (80 nM) was used as a mediator. The results are shown in Table 17.

### (5) Gluconobacter suboxydans

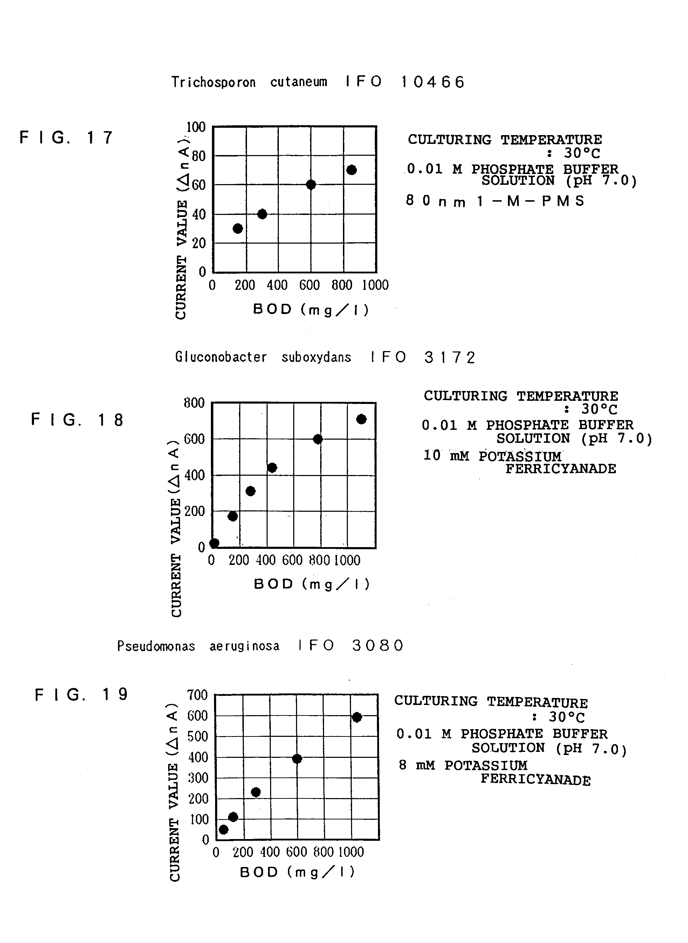

Gluconobacter suboxydans IF03172 was cultivated at 30 °C, and the experiment was conducted in the same manner as in the above (1). Potassium ferricyanade (10 mM) was used as a mediator. The results are shown in Fig. 18.

### (6) Pseudomonas aeruginosa

Pseudomonas aeruginosa IF010466 was cultivated at 30 °C, and the experiment was conducted in the same manner as in the above (1). Potassium ferricyanade (8 mM) was used as a mediator. The results are shown in Fig. 19.

The foregoing results reveal that BOD can be measured by the method of the present invention even if the various kinds of the microorganisms are used.

### Example 12

### Measurement of BOD in a solution containing a small amount of dissolved oxygen

A nitrogen gas was passed through a 0.01 M phosphate buffer solution for 30 minutes, and DCIP and 1-M-PMS were then added thereto such that the final concentration of each of DCIP and 1-M-PMS became 40 nM in 5 ml of the buffer solution. The same BOD sensor as that used in Example 1 was immersed in the mixture. A potential difference of +400 mV was applied thereto, and a BOD standard solution was added. An increase in the current value was measured while flowing a nitrogen gas onto the liquid surface in order to keep dissolved oxygen absent or present in the small amount in the buffer solution.

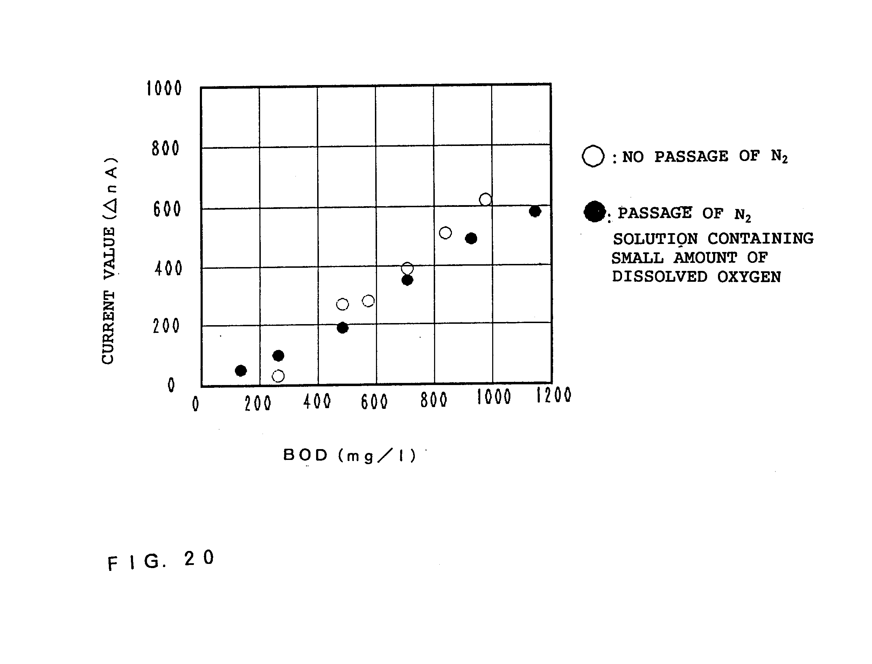

Approximately the same results were obtained whether the measurement was conducted while flowing the nitrogen gas or without flowing the nitrogen gas as usual, as shown in Fig. 20. Accordingly, it was shown that using the BOD sensor of the present invention, BOD can be measured with the solution containing the small amount of dissolved oxygen or with the solution free of dissolved oxygen.

### Example 13

### Other Example of a BOD sensor

Cells of L-GL3 which was obtained by the same manner as in Example 1 was immobilized on a metal surface of a cylindrical platinum electrode having a diameter of 3 mm with the use of agarose, a photo-crosslinkable polyvinyl alcohol (PVA-SbQ: polyvinyl alcohol stilbazole quaternized) or glutaraldehyde to produce a working electrode. BOD was measured using a BOD sensor comprising the working electrode, a counter electrode and a reference electrode.

### (1) Agarose

Agarose was heat-dissolved to a suitable concentration (for example, 2 %), and mixed with a suspension obtained by suspending the L-GL3 cells in the phosphate buffer solution. The thus-obtained agarose solution in which the cells were suspended was uniformly adhered to one end surface of the cylindrical platinum electrode having a diameter of 3 mm, and cooled to produce a working electrode.

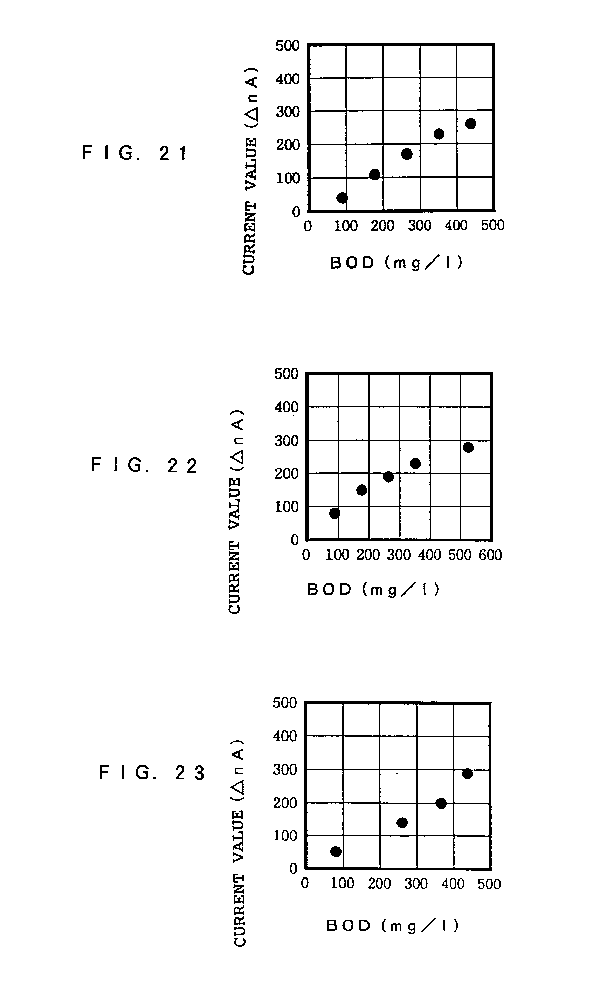

Using the working electrode, a counter electrode and a reference electrode, BOD was measured using the same measuring system as that used in Fig. 1. The above-mentioned BOD standard solution was used as the sample, and 100 nM 1-M-PMS was used as the mediator. The results are shown in Fig. 21.

### (2) PVA-SbQ

The L-GL3 suspension which was obtained by suspending the L-GL3 cells in the phosphate buffer solution was mixed with a suitable amount of the PVA-SbQ solution. The mixture was uniformly adhered to the metal surface of the cylindrical platinum electrode having a diameter of 3 mm, and incubated in a dark place at 37 °C for 3 hours. Thereafter, PVA-SbQ was crosslinked by being irradiated with a fluorescent lamp.

Using the working electrode, BOD was measured in the same manner as in the above (1) except that 1 mM potassium ferricyanade was used as the mediator. The results are shown in Fig. 22.

### (3) Glutaraldehyde

Bovine serum albumin (BSA) in a suitable amount was dissolved in the L-GL3 suspension which was obtained by suspending the L-GL3 cells in the phosphate buffer solution, and the mixture was uniformly adhered to the metal surface of the cylindrical platinum electrode having a diameter of 3 mm. The thus-obtained electrode was exposed to the vapor of a glutaraldehyde aqueous solution having a concentration of approximately 25 % for 20 minutes, and then rinsed three times with the phosphate buffer solution to produce a working electrode.

Using the working electrode, BOD was measured in the same manner as in the above (1) except that 80 nM 1-M-PMS was used as the mediator. The results are shown in Fig. 23.

The foregoing results reveal that BOD can be measured with the BOD sensor of the present invention even though the cells are immobilized on the metal electrode.

### Industrial Use

When the BOD sensor of the present invention is used, BOD can be measured without being influenced by the concentration of dissolved oxygen.

## Claims

1. A BOD sensor, comprising:
a microorganism electrode having an electrode made of metal or carbon and a membrane containing a microorganism cells which membrane is in contact with the electrode; and
a counter electrode.

2. A BOD sensor of claim 1 which further comprises a reference electrode.

3. A BOD sensor of claim 1 or 2, wherein the microorganism is a procaryote.

4. A method for measuring BOD, comprising the steps of:
immersing in a solution containing organic compounds a BOD sensor which comprises a working electrode having an electrode made of metal or carbon and a membrane containing a microorganism cells which membrane is in contact with the electrode, and a counter electrode; and
measuring a current that flows when a potential difference is applied between the working electrode and the counter electrode to measure the BOD of the solution.

5. A method of claim 4, further comprising the step of immersing a reference electrode in the solution, wherein the BOD of the solution is mesured by measuring a current that flows when a potential difference is applied between the working electrode and the reference electrode.

6. A method of claim 4 or 5, wherein the microorganism is a procaryote.

7. A method of any one of claims 4 to 6, wherein a mediator is added to the solution containing the organic substance and/or the microorganism membrane.

8. A method of claim 7, wherein the mediator is selected from the group consisting of 1-methoxy-5-methylphenazinium methylsulfonate, 2,6-dichloroindophenol and potassium ferricyanade.

9. A method of claim 7, wherein the mediator is added at a concentration of at least 10 nM.
